# EUROPEAN PATENT SPECIFICATION

(11) **EP 1 157 717 B1**
(45) Date of publication and mention of the grant of the patent: **12.01.2005**
(21) Application number: 00850092.8
(22) Date of filing: 26.05.2000
(51) Int. Cl.: A61N 1/39, A61B 5/0205

(54) **System for measuring and analysing Cardio-Pulmonary Resuscitation (CPR) parameters for use with and by an external defibrillator (AED)**
System zur Messung und Analyse von kardiopulmonaren Wiederbelebungs-Parametern zum Gebrauch mit einem externen Defibrillator
Système de mesure et d'analyse des paramètres de la réanimation cardio-pulmonaire destiné à être utilisé avec un défibrillateur externe

(43) Date of publication of application: 28.11.2001
(73) Proprietor: Laerdal Medical AS, 4001 Stavanger (NO)
(72) Inventor: Myklebust, Helge, 4025 Stavanger (NO); Eikeland, Harald, 4300 Sandnes (NO); Eftestol, Trygve, 4033 Forus (NO)
(74) Representative: Herbjörnsen, Rut

(56) References cited:
- EP-A- 0 756 878
- US-A- 4 273 114
- US-A- 4 619 265
- US-A- 5 544 661
- US-A- 5 853 292

## Description

The present invention relates to a system for measuring and analysing Cardio-Pulmonary-Resuscitation (CPR ) parameters for use with and by an external defibrillator, including a training defibrillator, according to the first part of Claim 1.

Such a device is known from EP-A-0 756 878.

The term external defibrillator covers all types of defibrillators that employ pads to be connected to the outside of the patient's chest. This also includes training defibrillators, the appearance and behaviour of which simulate a real defibrillator without being able to discharge defibrillator shocks. Training defibrillators are used with manikins during training and practice. As a comparison, an "internal defibrillator" or so-called implanted defibrillator is a device that is placed underneath the patient's skin, and which has electrodes connected directly to the heart muscle.

For years, the use of defibrillators in the case of sudden cardiac death has been recognised as the only curative treatment. Defibrillation involves the discharge of an electric pulse with relatively high energy (defibrillator shock) through electrodes connected to the patient's chest. Several designs of electrodes exist, but in the main they can be divided into two groups: "Paddles" are electrodes that are held manually on the chest. Adhesive electrodes or "Pads" are electrodes that are attached to the chest by using an adhesive, and which make use of a conducting polymer in order to establish good electric contact with the skin.

External defibrillators use the electrodes to discharge the electrical shock, to measure the patient's ECG, and to measure any impedance. The purpose of the impedance measurement is to determine the degree of electrical connection between the electrodes. If the impedance is close to zero, this would suggest a short circuit between the electrodes. In the opposite case, if the impedance is high, this would imply insufficient contact between the electrodes. Some types of defibrillators use impedance measurements in order to determine the voltage and time for discharging the defibrillator shock, so that the energy delivered to the patient is approximately equal to the desired energy.

Defibrillation may, by its very nature, involve a risk for those who treat the patient, if they touch the patient or in any other way come into contact with the electrodes. Therefore, the procedures for use include making sure everyone is clear before the shock is discharged. A defibrillator will employ voice messages in order to get the user and any assistants to follow procedures.

Traditionally, defibrillation has been carried out by highly trained personnel in hospitals. However, as automated defibrillators have developed over the last ten years and have become significantly easier to use, they have also come to be used outside of the hospitals, primarily by the ambulance services. There is also a clear tendency for defibrillators to be used by the laity before the ambulance reaches the patient. This refers especially to fire-fighters, police, guards and flight crews. Common to all of them is the fact that their profession is non-medical.

The trend today is that so-called patients at risk, for instance patients waiting for heart surgery, may have a defibrillator in their home, which can be operated by family members or others in the local environment in case of an emergency. Combined with giving the family CPR training, this ensures a high state of preparedness, in addition to an extra sense of security for the patient.

Unfortunately, attempts at resuscitation following sudden cardiac death are not always successful. When sudden cardiac death occurs, the outcome will among other things be dependent on all the links of the life-saving chain. These links are: Early notification of the support machinery, early CPR, early defibrillation and early advanced cardiac life support. The first treatment given to the patient is the most important. For the patient, it is of vital importance that the treatment not only be started as soon as possible, but also that the treatment is as effective and efficient as possible. In those cases where the heart does not start beating after the first three shocks given with a defibrillator, the treatment protocol prescribes one minute of CPR. The purpose of the CPR is to provide circulation of blood to the heart muscle, which increases the electrical activity in the heart, which in turn increases the probability of an electrical shock from a defibrillator being able to restart the heart. Thus, it is crucial for some patients that the CPR is administered effectively and correctly.

Operating an automated defibrillator is relatively simple, as today's defibrillators employ voice messages to instruct the user, and the apparatus does not normally have more than two control buttons. Performing CPR correctly is however fairly difficult for those who are inexperienced, as this calls for a command of both method and psychomotory skills. This despite the fact that the user has gone through training and practice.

It is becoming common practice, both in Norway and abroad, that a MECC (Medical Emergency Communication Centre) instructs the user in first aid, including CPR, pending the arrival of the ambulance. This assumes that there is an accessible telephone by the patient, preferably with a speaker function so as to let the user concentrate on the patient without having to hold on to the telephone. However, a shortcoming of this situation is the fact that the MECC does not receive concise and quantitative feed-back regarding the events taking place, but have to interpret the situation as best they can based on the information received over the telephone.

Using a defibrillator and performing CPR on people generally presupposes that the user has gone through training and practice.

Today, all CPR training and practice includes the use of special manikins. These manikins have been constructed so as to allow inflations and chest compressions to be performed roughly in the same manner as on a lifeless person. Such a device is disclosed in US-A-5 853 292. Most manikins have been fitted with a number of sensors that among other things register lung inflations and chest compressions. This registration is used to provide visual feedback regarding the performance, as well as for generating a report on the performance with a view to certification in accordance with the guidelines. When defibrillator training is carried out, this may happen in two alternative ways: If the practice is non-interactive, the defibrillator or the training defibrillator will be equipped with simple adhesive electrodes that are attached to the manikin's chest. Normally, there will be no interaction or communication between the manikin and the training defibrillator.

Interactive practice will normally entail the use of a real defibrillator and a manikin that for defibrillating purposes behaves as a human being. The general design for such interactive use has the manikin equipped with an internal load resistance connected to the connection points on the manikin's chest. The patient cable from the defibrillator has been designed for connection to the connection points. The load resistance will indicate to the defibrillator that it is connected to a patient. Further, the manikin will be provided with a signal transmitter that simulates the electrical activity of the heart (ECG). The signal transmitter is connected to the load resistance, to ensure that this signal is also available to the defibrillator. Finally, the load resistance is designed in such a way as to enable it to absorb the defibrillator shocks. A sensor connected in series with the load resistance is normally designed to be able to influence the signal transmitter.

The object of the present invention is to provide a system that enables a person with limited knowledge and skills to perform CPR correctly, efficiently and more effectively, so as to give the patient better treatment and thereby a greater chance of survival.

It is also an object of the present invention to provide a system such that training and practice in the use of defibrillators of the type that measures and analyses CPR parameters, with accompanying voice messages, may be as realistic as possible.

This is provided by means of a system of the type mentioned at the beginning, the characteristics of which are apparent from Claim 1. Further features of the present invention are apparent from the remaining, dependent Claims.

The information provided by the present invention is used by a defibrillator in order to give corrective voice messages to the user. In addition, this information may be transmitted to a medical emergency communication centre (MECC) that will interpret the situation and guide the user during the resuscitation attempt. Moreover, the information collected will contribute towards documenting the treatment, which in turn is important with regards to quality assurance and treatment optimisation.

The following will explain the invention in more detail, with reference to the accompanying drawings, in which
- Fig. 1: schematically shows a person connected to a defibrillator;
- Fig. 2: shows a schematic circuit diagram of the patient connection;
- Fig. 3: schematically shows the system according to the invention connected to an emergency centre, and a potential display of the information transmitted to the centre;
- Fig. 4: shows a schematic block diagram of the system according to the invention;
- Fig 5: shows signals measured during resuscitation of a pig, where the upper curve shows the signal proportionally with the impedance change, and the lower curve shows the signal proportionally with the depression of the chest; and

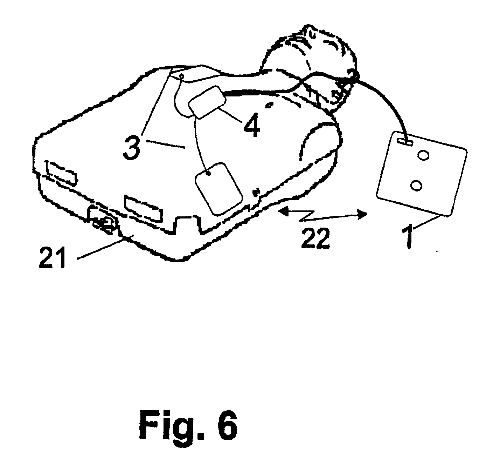

- Fig 6: shows a defibrillator or a training defibrillator connected to a manikin, where the information regarding the CPR performance is communicated between the units.

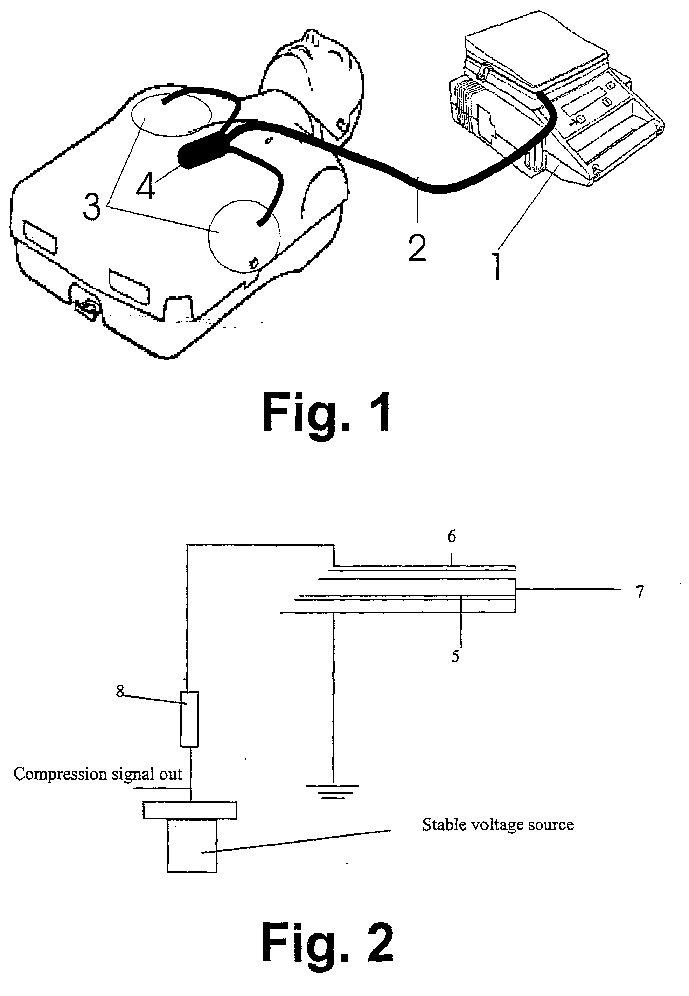

Figure 1 shows a pressure sensitive pad 4 and electrodes 3 attached to the patient's chest. The top of the pad 4 may be provided with instructions (written text and figures) that tell the user how to position the pad 4, for instance in relation to the patient's nipples. The pad 4 represents an indication of where the hands of the life-saver should be placed for chest compression, and it also functions as a force indicator. The pad 4 will transmit an electrical signal to the defibrillator 1, corresponding to the applied pressure (force). The pad 4 is placed over the patient's heart and attached to the patient's chest in an electrically conducting manner, and forms the signal reference (zero, ground) for the defibrillator's measurements of the ECG signal. This connection improves the quality of the ECG signal, as the charge between the defibrillator and the patient may be balanced through zero instead of through the measuring electrodes, thus reducing the signal interference.

Figure 2 shows the principles of the pressure pad, which consists of an inner core 7 made from electrically conductive foam or electrically conductive silicone or other practical force sensors. The side facing the patient's body consists of a metal foil 5, in order to ensure that the current is distributed evenly across the area, with an electrically conductive adhesive against the patient's skin. The top of the pressure pad consists of a metal foil 6 with said printed instructions and figures, which foil ensures a constant current.

As any pressure on the pad will alter the impedance through the pad, an approximately constant impressed current will give a variable voltage to the processing unit 20 in the defibrillator 1. In its simplest form, an approximately constant current can be achieved by means of series impedance that is significantly greater than the impedance through the pressure pad.

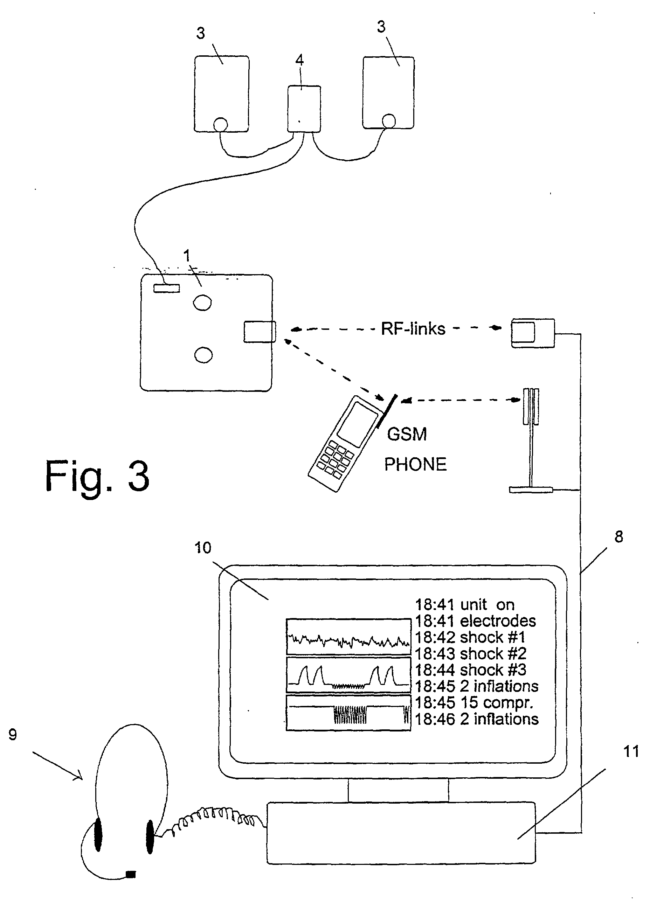

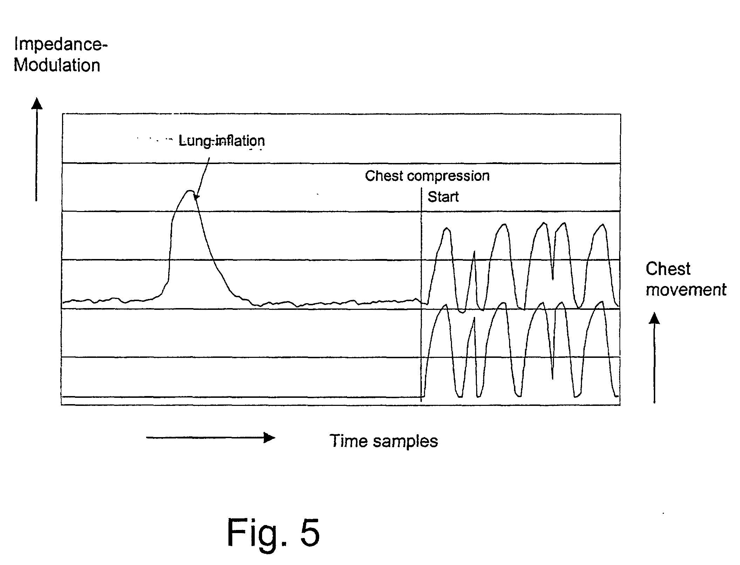

Standard defibrillator electrodes 3 are used by the impedance system 13 in order to measure the impedance of the thorax (chest), by the ECG-measuring system 12, and by the high voltage system 14 in order to deliver the electrical shock to the patient. The impedance of the thorax is normally measured by supplying an approximately constant alternating current between the defibrillator electrodes 3 and at the same time measuring the alternating voltage between the electrodes 3, which is proportional to the impedance (impedance times current input equals the voltage). The preferred frequency is 30 kHz, but frequencies from 0.1 kHz to 100 kHz may be used. 30 kHz is preferred because the impedance that can be measured at this frequency is close to the impedance that determines the current during discharge of the defibrillator shock. A change of the impedance signal, that is a change in the alternating voltage measured between the electrodes at an approximately constant alternating current between the electrodes, is used to calculate CPR parameters. On a lung infation, the impedance of the thorax increases through the current path being made longer. On chest compression, experiments on pigs show the impedance of the thorax increasing here as well, however the increase is significantly smaller than during lung inflations. When CPR is performed according to the guidelines, 2 inflations will be given, each lasting 1.5 to 2 seconds, followed by a short break as the life-saver changes position, then 15 chest compressions at a rate of approximately 100/minute, again followed by a short break as the life-saver changes position in order to administer lung inflations. By utilising this pattern of sequential actions, where the sequential actions are distinctively different, an analysis of the impedance signal in the defibrillator 1 will enable the following CPR parameters to be deduced: Inflation occurrences and inflation time, the occurrence of chest compressions and compression rate, see Figure 3, in which a screen 10 shows ECG, inflation time and compressions over time respectively; and Figure 5, which illustrates how lung inflations and chest compressions affect the impedance between the electrodes.

The defibrillator 1 also has a driver and sensor electronics for the pressure sensitive pad (the electrode) 4, the defibrillator 1 being connected to the electrodes 3 and said pad 4 via a cable 2. The signal from the pressure sensitive pad 4 is amplified and filtered before being fed to an analogue/digital converter for further analysis and derivation of CPR data, such as compression rate and compression force.

The defibrillator 1 contains an algorithm, e.g. a table of standardised parameters, which, based on a comparison with measured CPR parameters, activates a speaking device that informs the user of any mistakes in the CPR, and which contributes towards increasing the quality of the CPR by supplying information regarding the correct magnitude of chest compressions, inflation time, change-over from inflation to chest compression and back to inflation etc.

The algorithms described herein may be in the form of computer programmes that compare the measured parameters with standardised methods of treatment according to ILCOR and AHA respectively.

According to the instructions, the defibrillator is only to be used on lifeless persons. The treatment consists of alternating defibrillation (shocks) and CPR according to the following pattern:
Administer three shocks, then one minute of CPR, then three new shocks etc. CPR consists of inflating twice, then performing 15 chest compressions, followed by inflating twice again etc.

The following overview shows typical situations that the defibrillator can monitor and attempt to assist the user in, by means of voice messages:

| **Situation** | **Voice message** |
|---|---|
| Inflation does not commence | "Inflate twice" or "blow now" or "blow twice" |
| Inflation does not occur despite above messages | "Clear the respiratory passages by bending the head back and lifting the chin" or "remember to pinch the nose shut" |
| Inflation time < 1.5 sec | "Take longer" or "not so fast" |
| The second inflation is too long in coming | "Once more" |
| Inflation re-commences too late following 15 compressions | "Blow now" or "blow twice" |
| Compressions do not commence | "Press down between the nipples" |
| Compression rate ≠ 100 +/- 10 | "Press faster" / "Not so fast" |
| Compressions re-commence too late following the second inflation | "Start pressing" or "press down on the chest" or "press down 15 times" |

The defibrillator can also start an audible metronome as a guide to the rate of compressions. This may be desirable if the chest compressions interfere with the ECG signal, as a set compression rate makes it easier to filter out the interference, which will keep within a narrow range.

The defibrillator 1 may also contain an algorithm that calculates the median frequency and the spectral flatness of the filtered ECG signal, and combines the two into a vector, the positive change in magnitude by time of which is compared to a threshold value. If the calculated parameter lies below the threshold value, the defibrillator 1 will recommend a small increase in the compression force. The threshold value is set so as to ensure the quickest possible positive change.

The defibrillator 1 may also contain an algorithm that calculates the median frequency and the spectral flatness of the filtered ECG signal, and combines the two into a vector, the magnitude of which is compared to a threshold value. When the calculated vector magnitude lies below the threshold value, the defibrillator 1 will recommend CPR, or else it will recommend defibrillation. The threshold value is set so as to ensure the highest possible probability of returning to spontaneous circulation.

The data collected, such as ECG, ventilation data, CPR information and other measured data regarding the condition of the patient and the activities carried out on the patient during treatment may, instead of being transferred to a communication centre or in addition to the transfer, be stored in a memory 19, from which the data may later be retrieved via e.g. a PC for further evaluation of the CPR that has been performed.

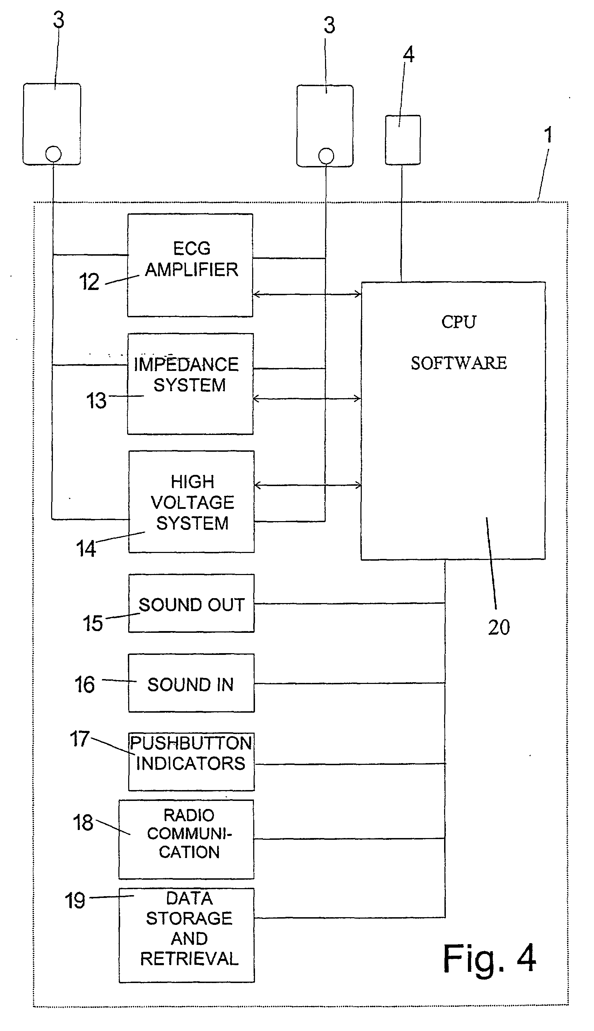

Further, the defibrillator 1 may be provided with a transmitter-receiver device 18 that, when the apparatus is switched on, uses a radio frequency link to connect to and log onto a computer 11 (see Fig. 3) at the MECC via e.g. a mobile telephone or a modem. After connecting, the defibrillator 1 will identify itself and continuously transmit various data such as the ECG signal, chest compression data and inflation data, which is displayed on a screen 10 at the emergency centre. Moreover, a speech connection will be established with the supervisory centre, in order to allow the defibrillator 1 user to communicate with the emergency centre operator 9 via a microphone 16 and a speaker 15 (see Fig 4). The defibrillator will also be able to receive instructions from the emergency centre. In case of such voice communication, the request for speech, e.g. from the emergency centre operator, will imply a barring of all other messages from the defibrillator, with the exception of the messages that control the operator's or patient's safety.

This communication will enable the operator to interpret and guide the lifesaving operation. Further, the presence of the MECC operator through voice communication will help make the user feel more secure as he or she is about to perform particularly critical, life-saving first aid. The system may, as a safety precaution, be set up in such a way that communication can only be shut down by the emergency centre operator.

By providing the manikin 21 with sensors that register CPR performance and a micro processor that processes and stores that CPR performance, realistic training will be possible through the manikin's micro processor communicating the CPR parameters to the micro processor in the defibrillator. Such communication 22 may take place by means of a cable between the units or by wireless communication if the defibrillator is equipped with bi-directional radio or infrared communication.

## Claims

1. An external defibrillator for use on live human beings, comprising at least one pair of electrodes for providing electric pulses to a patient's chest, and reading electrical signals from the patient's chest, and voice prompting means for instructing a life-saver on the recommended Cardio-Pulmonary-Resuscitation, CPR,
said defibrillator being **characterized in that** it comprises means (13, 20) for measuring and analysing CPR parameters, said means comprising receiving means for receiving information regarding chest compressions from at least one chest compression sensor (4) and information regarding lung inflation from at least one lung inflation sensor (3), said sensors (3, 4) being adapted to be placed externally on the patient, and processing means (13, 20) for deriving, from information from the sensors (3,4), CPR parameters that describe the CPR performance and comparing the CPR parameters with corresponding parameters that express the guidelines for recommended CPR and for generating voice messages based on the difference between performed CPR and recommended CPR to instruct the life-saver.

2. An external defibrillator according to claim 1, wherein the chest compression sensor (4) comprises a pressure-sensitive pad arranged to indicate where the life-saver's hands should be placed for chest compression, and also to provide an electrical signal to the defibrillator corresponding to the force applied to the sensor by the life-saver, the defibrillator comprising calculating means (20) for calculating CPR data.

3. An external defibrillator according to claim 1 or 2, wherein the lung inflation sensor (3) comprises means for providing a current between the pair of electrodes (3) and means for measuring the impedance between the electrodes (3) and calculating lung inflation characteristics as a function of the impedance between the electrodes.

4. An external defibrillator according to any one of the preceding claims wherein the sensors are arranged to be applied to the patient by means of an adhesive.

5. An external defibrillator according to claim 1, wherein the defibrillator (1) is provided with a transmission device (18) for establishing a radio link to an emergency centre via a modem, GSM telephone or similar when the defibrillator (1) is activated in normal mode, where the emergency centre is equipped with a display unit (10) that shows the CPR parameters derived at the resuscitation site, and
further comprising means for establishing, in addition to a data transfer link, a speech connection via a microphone and a speaker on the resuscitation site and a microphone (16) and auditory unit (15) at the emergency centre, in order to enable an operator (9) at the emergency centre to intervene and direct the attempt at resuscitation during the critical phases following the electrical shocks.

6. An external defibrillator according to claim 5, wherein the defibrillator (1) is able to assume a training mode in which it is arranged to receive information regarding chest compressions and lung inflations from sensors in a manikin, where communication with the manikin is wireless or takes place through a cable, and where the defibrillator (1) further comprises communication means for communicating directly with a computer that is operated by an instructor, in order for these to simulate the connection with an emergency centre.

7. An external defibrillator according to claim 2, wherein the top (6) of the pressure sensitive pad is provided with instructions regarding where to place the electrode, for instance in relation to the patient's nipples.

8. An external defibrillator according to claim 7, wherein the pressure sensitive electrode is arranged to form the signal reference (zero, ground) for the defibrillator's measurements of the ECG signal, so as to improve the signal quality by reducing the signal interference.

9. An external defibrillator according to claim 6, wherein the lung inflation sensor (3) is an impedance measuring system, comprising means for delivering an approximately constant alternating current is delivered to the patient, the current path running between the electrodes (3) of the defibrillator (1), which are attached to the chest of the patient, the defibrillator comprising means for measuring a change in the voltage between the electrodes (3), said voltage being generated by the current through the impedance between the electrodes (3), said change being caused by lung inflations and chest compressions.

10. An external defibrillator according to claim 9, wherein the defibrillator comprises processing means (20) for analysing the signal from the impedance measuring system in order to deduce inflation occurrences and inflation time, occurrence of chest compressions and chest compression rate, as a lung inflation increases the impedance and a chest compression increases the chest impedance, and wherein a lung inflation is identified as distinct from a chest compression through the characteristic differences in signal changes caused by chest compressions and lung inflations respectively.

11. An external defibrillator according to claim 9, wherein an alternating current with a frequency of between 0.1 and 100 kHz is used as an excitation signal, the frequency preferably being 30 kHz.

12. An external defibrillator according to claim 1, wherein the defibrillator (1) contains an algorithm that calculates the median frequency and the spectral flatness of the filtered ECG signal, and combines the two into a vector, the magnitude of which constitutes a parameter that is compared to a threshold value, and wherein the defibrillator, when this value lies below the threshold value, will recommend CPR, or else it will recommend defibrillation.

## Patentansprüche

1. Ein externer Defibrillator zur Verwendung am lebenden Menschen, mit mindestens einem Paar von Elektroden zum Bereitstellen von elektrischen Impulsen für eine Patientenbrust, und zum Lesen von elektrischen Signalen von der Patientenbrust, und einer Sprachaufforderungseinrichtung zum Anweisen eines Lebensretters bezüglich empfohlener Herz-Kreislauf-Wiederbelebung, CPR,
wobei der Defibrillator **dadurch gekennzeichnet ist, dass** er eine Einrichtung (13, 20) umfasst, zum Messen und Analysieren von CPR-Parametern, wobei die Einrichtung eine Empfangseinrichtung zum Empfangen von Information bezüglich Brustkompressionen von mindestens einem Brustkompressionssensor (4) und Information bezüglich einer Lungeninflation von mindestens einem Lungeninflationssensor (3) umfasst, wobei die Sensoren (3, 4) dazu abgepasst sind, extern am Patienten angeordnet zu werden, und eine Verarbeitungseinrichtung (13, 20) zum Ableiten, von der Information von den Sensoren (3, 4), von CPR-Parametern, die die CPR-Leistung beschreiben, und zum Vergleichen der CPR-Parameter mit entsprechenden Parametern, die Richtlinien für empfohlene CPR beschreiben, und zum Erzeugen von Sprachnachrichten, um den Lebensretter basierend auf dem Unterschied zwischen der durchgeführten CPR und der empfohlenen CPR anzuweisen.

2. Ein externer Defibrillator nach Anspruch 1, wobei der Brustkompressionssensor (4) ein druckempfindliches Kissen umfasst, dass dazu angeordnet ist, anzuzeigen, wo die Hände des Lebensretters für eine Brustkompression angeordnet sein sollten, und um weiter ein elektrisches Signal an den Defibrillator entsprechend der an den Sensor durch den Lebensretter aufgebrachten Kraft zu liefern, wobei der Defibrillator eine Berechnungseinrichtung (20) zum Berechnen von CPR-Daten umfasst.

3. Ein externer Defibrillator nach Anspruch 1 oder 2, wobei der Lungeninflationssensor (3) eine Einrichtung umfasst, um einen Strom zwischen dem Paar von Elektroden (3) bereitzustellen, und eine Einrichtung zum Messen der Impedanz zwischen den Elektroden (3) und zum Berechnen von Lungeninflationscharakteristiken als eine Funktion der Impedanz zwischen den Elektroden.

4. Ein externer Defibrillator nach einem der vorhergehenden Ansprüche, wobei die Sensoren dazu angeordnet sind, mittels eines Haftstoffes an dem Patienten angebracht zu werden.

5. Ein externer Defibrillator nach Anspruch 1, wobei der Defibrillator (1) mit einer Übertragungsvorrichtung (18) ausgestattet ist, um eine Funkverbindung mit einem Notfallzentrum über ein Modem, GSM-Telefon oder ähnliches einzurichten, wenn der Defibrillator (1) in einem Normalmodus aktiviert wird, wobei das Notfallzentrum mit einer Anzeigeeinheit (10) ausgestattet ist, die die an dem Wiederbelebungsort abgeleiteten CPR-Parameter anzeigt, und
weiter mit einer Einrichtung zum Einrichten, zusätzlich zu der Datenübertragungsverbindung, einer Sprachverbindung über ein Mikrophon und einen Lautsprecher an dem Wiederbelebungsort, und einem Mikrophon (16) und einer Höreinheit (15) an dem Notfallzentrum, um einen Operator (9) am Notfallzentrum in die Lage zu versetzen, einzugreifen und den Versuch der Wiederbelebung während der kritischen Phasen nach den elektrischen Schocks zu leiten.

6. Ein externer Defibrillator nach Anspruch 5, wobei der Defibrillator (1) in der Lage ist, einen Trainingsmodus einzunehmen, in dem er dazu angeordnet ist, Information bezüglich Brustkompressionen und Lungeninflationen von Sensoren in einer Puppe zu empfangen, wobei die Kommunikation mit der Puppe drahtlos ist oder über ein Kabel stattfindet, und wobei der Defibrillator (1) weiter eine Kommunikationseinrichtung zum direkten Kommunizieren mit einem Computer umfasst, der durch einen Instruktor betrieben wird, um dafür die Verbindung mit einem Notfallzentrum zum simulieren.

7. Ein externer Defibrillator nach Anspruch 2, wobei die Oberseite (6) des druckempfindlichen Kissens mit Instruktionen ausgestattet ist, wo die Elektrode anzuordnen ist, beispielsweise in Bezug auf die Brustwarzen des Patienten.

8. Ein externer Defibrillator nach Anspruch 7, wobei die druckempfindliche Elektrode dazu angeordnet ist, den Signalbezug (Null, Masse) für die Messungen des Defibrillators des EKG-Signals zu bilden, um so die Signalqualität durch Reduzieren der Signalinterferenz zu verbessern.

9. Ein externer Defibrillator nach Anspruch 6, wobei der Lungeninflationssensor (3) ein Impedanzmesssystem mit einer Einrichtung zum Liefern eines ungefähr konstanten Wechselstroms ist, wobei der Strompfad zwischen den an der Brust des Patienten angeordneten Elektroden (3) des Defibrillators (1) verläuft, der Defibrillator eine Einrichtung zum Messen einer Änderung der Spannung zwischen den Elektroden (3) umfasst, wobei die Spannung durch den Strom über die Impedanz zwischen den Elektroden (3) erzeugt wird, und wobei die Änderung durch Lungeninflationen und Brustkompressionen bewirkt wird.

10. Ein externer Defibrillator nach Anspruch 9, wobei der Defibrillator eine Verarbeitungseinrichtung (20) umfasst, um das Signal von dem Impedanzmesssystem zu analysieren, um Inflationserscheinungen und eine Inflationszeit, ein Auftreten von Brustkompressionen und eine Brustkompressionsrate abzuleiten, wenn eine Lungeninflation die Impedanz erhöht und eine Brustkompression die Brustimpedanz erhöht, und wobei eine Lungeninflation als unterschiedlich von einer Brustkompression über die charakteristischen Unterschiede von Signaländerungen durch Brustkompressionen bzw. Lungeninflationen identifiziert wird.

11. Ein externer Defibrillator nach Anspruch 9, wobei ein Wechselstrom mit einer Frequenz von 0,1 und 100 kHz als Anregungssignal verwendet wird, wobei die Frequenz vorzugsweise 30 kHz ist.

12. Ein externer Defibrillator nach Anspruch 1, wobei der Defibrillator (1) einen Algorithmus umfasst, der die mittlere Frequenz und die spektrale Flachheit des gefilterten EKG-Signals berechnet und die zwei in einem Vektor kombiniert, dessen Größe einen Parameter darstellt, der mit einem Schwellwert verglichen wird, und wobei der Defibrillator dann, wenn dieser Wert unter dem Schwellwert liegt, zu einer CPR raten wird, oder andernfalls eine Defibrillation anraten wird.

## Revendications

1. Défibrillateur externe destiné à être utilisé sur des êtres humains vivants, comprenant au moins une paire d'électrodes pour envoyer des impulsions électriques au thorax d'un patient, et pour relever des signaux électriques du thorax du patient, et un moyen de guidage vocal pour donner à un secouriste des instructions concernant la réanimation cardio-respiratoire (RCP) recommandée,
ledit défibrillateur étant **caractérisé en ce qu'**il comprend un moyen (13, 20) servant à mesurer et à analyser des paramètres RCP, ledit moyen comprenant un moyen de réception destiné à recevoir des informations concernant les compressions thoraciques provenant d'au moins un capteur (4) de compressions thoraciques et des informations concernant l'insufflation des poumons provenant d'au moins un capteur (3) d'insufflation des poumons, lesdits capteurs (3, 4) étant adaptés pour être placés extérieurement sur le patient, et un moyen de traitement (13, 20) pour donner, à partir des informations provenant des capteurs (3, 4), des paramètres RCP qui décrivent les performances RCP et pour comparer les paramètres RCP avec des paramètres correspondants qui expriment les directives de la RCP recommandée et pour produire des messages vocaux basés sur la différence entre la RCP effectuée et la RCP recommandée afin de guider le secouriste.

2. Défibrillateur externe selon la revendication 1, dans lequel le capteur (4) de compressions thoraciques comprend une pastille sensible à la pression adaptée pour indiquer où les mains du secouriste doivent se placer pour la compression du thorax, et aussi pour fournir un signal électrique au défibrillateur qui correspond à la force appliquée au capteur par le secouriste, le défibrillateur comprenant un moyen de calcul (20) servant à calculer des données RCP.

3. Défibrillateur externe selon la revendication 1 ou 2, dans lequel le capteur (3) d'insufflation des poumons comprend un moyen servant à faire circuler un courant entre la paire d'électrodes (3) et un moyen servant à mesurer l'impédance entre les électrodes (3) et à calculer les caractéristiques d'insufflation des poumons en fonction de l'impédance entre les électrodes.

4. Défibrillateur externe selon l'une quelconque des revendications précédentes, dans lequel les capteurs sont adaptés pour être appliqués au patient au moyen d'un adhésif.

5. Défibrillateur externe selon la revendication 1, dans lequel le défibrillateur (1) est pourvu d'un dispositif d'émission (18) pour établir une liaison radio avec un centre de secours via un modem, un téléphone GSM ou appareil similaire lorsque le défibrillateur (1) est activé en mode normal, le centre de secours étant équipé d'une unité d'affichage (10) qui montre les paramètres RCP obtenus sur le lieu de la réanimation, et comprenant en outre un moyen servant à établir, en plus d'une liaison de transfert de données, une connexion vocale via un microphone et un haut-parleur sur le lieu de la réanimation et un microphone (16) et une unité auditive (15) dans le centre de secours, pour permettre à un opérateur (9) du centre de secours d'intervenir et de diriger la tentative de réanimation au cours des phases critiques qui suivent les chocs électriques.

6. Défibrillateur externe selon la revendication 5, dans lequel le défibrillateur (1) est apte à prendre un mode d'entraînement dans lequel il est adapté pour recevoir des informations concernant les compressions thoraciques et l'insufflation des poumons provenant de capteurs présents dans un mannequin, où la communication avec le mannequin est sans fil ou bien se fait au moyen d'un câble, et où le défibrillateur (1) comprend en outre un moyen de communication permettant de communiquer directement avec un ordinateur qui est utilisé par un instructeur, afin que ceux-ci simulent la connexion avec un centre de secours.

7. Défibrillateur externe selon la revendication 2, dans lequel le dessus (6) de la pastille sensible à la pression comporte des instructions concernant le positionnement de l'électrode, par exemple par rapport aux mamelons du patient.

8. Défibrillateur externe selon la revendication 7, dans lequel l'électrode sensible à la pression est adaptée pour former la référence du signal (zéro, masse) pour les mesures du signal ECG du défibrillateur, afin d'améliorer la qualité du signal en réduisant l'interférence des signaux.

9. Défibrillateur externe selon la revendication 6, dans lequel le capteur (3) d'insufflation des poumons est un système de mesure d'impédance, comprenant un moyen servant à fournir un courant alternatif à peu près constant délivré au patient, le chemin du courant se situant entre les électrodes (3) du défibrillateur (1), qui sont fixées au thorax du patient, le défibrillateur comprenant un moyen servant à mesurer une variation de la tension entre les électrodes (3), ladite tension étant produite par le courant circulant dans l'impédance présente entre les électrodes (3), ladite variation étant provoquée par les insufflations des poumons et par les compressions thoraciques.

10. Défibrillateur externe selon la revendication 9, dans lequel le défibrillateur comprend un moyen de traitement (20) servant à analyser le signal provenant du système de mesure d'impédance afin de déduire les occurrences des insufflations et le temps d'insufflation, l'occurrence des compressions thoraciques et le taux de compression thoracique, comme une insufflation des poumons augmente l'impédance et une compression thoracique augmente l'impédance du thorax, et dans lequel une insufflation des poumons est identifiée comme étant distincte d'une compression thoracique par les différences de caractéristiques des variations du signal provoquées respectivement par les compressions thoraciques et les insufflations des poumons.

11. Défibrillateur externe selon la revendication 9, dans lequel on utilise un courant alternatif ayant une fréquence comprise entre 0,1 et 100 kHz comme signal d'excitation, la fréquence valant de préférence 30 kHz.

12. Défibrillateur externe selon la revendication 1, dans lequel le défibrillateur (1) contient un algorithme qui calcule la fréquence moyenne et la planéité spectrale du signal ECG filtré, et combine les deux en un vecteur, dont l'amplitude constitue un paramètre qui est comparé à une valeur seuil, et dans lequel le défibrillateur, lorsque cette valeur se trouve sous la valeur seuil, recommande la RCP, sinon il recommande la défibrillation.
